# EUROPÄISCHE PATENTSCHRIFT

(11) **EP 1 214 950 B1**
(45) Veröffentlichungstag und Bekanntmachung des Hinweises auf die Patenterteilung: **24.01.2007**
(21) Anmeldenummer: 01129913.8
(22) Anmeldetag: 15.12.2001
(51) Int. Cl.: A61L 17/14, A61L 17/10

(54) **Nahtmaterial für die Chirurgie, Verfahren zu seiner Herstellung und Verwendung**
Surgical suture material, method of its manufacture and its use
Matériaux de suture chirurgicales et procédé pour sa fabrication et son utilisation

(30) Priorität: 16.12.2000 DE 10062881
(43) Veröffentlichungstag der Anmeldung: 19.06.2002
(73) Patentinhaber: DEUTSCHE INSTITUTE FÜR TEXTIL- UND FASERFORSCHUNG STUTTGART Stiftung des öffentlichen Rechts, 73770 Denkendorf (DE)
(72) Erfinder: Müller, Erhard, 70565 Stuttgart (DE); Oberhoffner, Sven, Dr., 71384 Weinstadt (DE); Planck, Heinrich, 72622 Nürtingen (DE)
(74) Vertreter: Patentanwälte Ruff, Wilhelm, Beier, Dauster & Partner

(56) Entgegenhaltungen:
- EP-A- 0 258 749
- EP-A- 0 608 139
- EP-A- 0 739 636
- EP-A- 0 908 142
- EP-A- 1 074 270
- DE-A- 19 641 334
- US-A- 5 854 383

## Beschreibung

Die Erfindung betrifft Nahtmaterial für die Chirurgie, Verfahren zu seiner Herstellung und seine Verwendung.

Bei der chirurgischen Behandlung von Verletzungen oder Erkrankungen im Bereich der Humanmedizin oder Veterinärmedizin ist eine leichte Handhabbarkeit von Nähfäden von wesentlicher Bedeutung für eine exakte Nahtlegung sowie deren Haltbarkeit und komplikationslose Heilung nach dem Eingriff.

Es wurden verschiedene chirurgische Nähfäden aus natürlichen und synthetischen Materialien entwickelt, die zur Verbesserung ihrer Gebrauchseigenschaften modifiziert sind. So ist es beispielsweise bekannt, mehrfädiges Nahtmaterial mit Fettsäuresalzen zu beschichten.

Bekannte Beschichtungen weisen jedoch noch Mängel im Durchzugs- und Verknotungsverhalten auf. Metallsalzbeschichtungen neigen bei der Verwendung zu unerwünschter Staub- oder Flockenbildung. Solche Beschichtungsmaterialien unterscheiden sich auch in ihrem physiologischen Verhalten, etwa in ihrer biologischen Abbaubarkeit, von den Nahtmaterialien, auf die sie aufgebracht sind. Ferner sind die Verfahren zur Herstellung beschichteter Nähfäden sehr aufwendig und kompliziert.

US 584383 beschreibt ein resorbierbares, segmentiertes Copolymer zur Verwendung als Nahtmaterial, das gebildet ist aus einem Prepolymer auf Basis von Trimethylencarbonat, Glykolid und Caprolacton, das mit Glykolid copolymerisiert ist.

DE 19641334 offenbart ein Triblockterpolymer mit Hartsegment- und Weichsegmentanteilen zur Herstellung von medizinischen Produkten.

EP 0908142 beschreibt niedermolekulare Homopolymere und Copolymere als Beschichtung für geflochtenes Nahtmaterial.

EP 0258749 betrifft Beschichtungszusammensetzungen für Nahtmaterial auf Basis von Hydrogelen aus Blockcopolymeren und Erdalkalimetall.

Die Erfindung stellt sich daher die Aufgabe, ein Nahtmaterial zur Verfügung zu stellen, das die erkannten Nachteile von Materialien aus dem Stand der Technik überwindet, das einfach und kostengünstig mit üblichen Geräten herzustellen und in der Chirurgie mit Vorteil zu verwenden ist.

Diese Aufgabe wird gelöst durch ein Nahtmaterial für die Chirurgie bestehend aus einem oder mehreren Filamenten und ausgebildet mit einer Beschichtung, dadurch gekennzeichnet, dass die Beschichtung mindestens teilweise aus einem wachsartigen bioresorbierbaren Polymer besteht, das im wesentlichen aus einem statistischen Terpolymer mit völlig amorpher Struktur gebildet ist, das Terpolymer unter Verwendung von Glykolid, ε-Caprolacton und Trimethylencarbonat gebildet ist, insbesondere aus diesen besteht, und im Terpolymer Glykolid in einem Anteil von 5 bis 50 Gew.-%, ε-Caprolacton in einem Anteil von 5-90 Gew.-% und Trimethylencarbonat in einem Anteil von 5-90 Gew.-% vorhanden ist.

Das zur Beschichtung gemäss der Erfindung eingesetzte rohe Nahtmaterial kann aus den Fachleuten bekannten natürlichen oder synthetischen Filamenten auf ebenfalls bekannte Weise ausgebildet sein. Beispielsweise können Monofilamente, Multifilamente oder Kombinationen von Monofilamenten und Multifilamenten unterschiedlicher chemischer Natur zu Nahtmaterial ausgebildet sein. Als geeignete synthetische Filamente sind bioverträgliche resorbierbare oder nicht resorbierbare Polymere wie Homopolymere, Copolymere, Terpolymere oder Kombinationen davon zu nennen. Als Beispiele sind Polymere ausgehend von biokompatiblen Monomeren wie Glykolsäure, Glykoliden, Lactiden, Dioxanonen, Lactonen und anderen anzuführen.

Mit besonderem Vorteil kann beim Nahtmaterial das Terpolymer unter Verwendung von Glykolid, e-Caprolacton und Trimethylencarbonat gebildet sein, insbesondere aus diesen bestehen. Es kann anstelle eines dimeren Glykolids Glykolsäure verwendet sein, wodurch eine Regulierung des Molekulargewichts möglich ist. Im Terpolymer ist Glykolid in einem Anteil von 5 bis 50 Gew.-%, ε-Caprolacton in einem Anteil von 5 bis 90 Gew.-% und Trimethylencarbonat in einem Anteil von 5 bis 90 Gew.-% vorhanden. Die Gewichtsanteile der Komponenten Trimethylencarbonat, ε-Caprolacton und Glykolid sind so gewählt, dass sie zusammengenommen 100 Gew.-% des Terpolymers ausmachen. Ausserdem kann im Terpolymer Trimethylencarbonat und e-Caprolacton in einem Gewichtsverhältnis zwischen 95 : 5 und 5 : 95, insbesondere in einem Gewichtsverhältnis von 30 : 70 bis 70 : 30, bevorzugt von 50 : 50 vorhanden sein.

Es wurde überraschend gefunden, dass ein hochmolekulares Polymer als Beschichtungsmaterial für chirurgische Nähfäden zur Verbesserung der Nahtmaterialeigenschaften und insbesondere der Verknotungseigenschaften geeignet ist. Als günstig beeinflusste Eigenschaften sind beispielsweise Knotenlauf, Knotensitz, Knotenhaltbarkeit und Knotensicherheit zu nennen.

Ein höherer Anteil, von 5 bis 90 Gew.-%, an Caprolacton im Terpolymer weist einen günstigen Einfluss auf den Knotenlauf des damit beschichteten chirurgischen Nahtmaterials auf. In einer bevorzugten Ausführungsform können Glykolid, Caprolacton und Trimethylencarbonat in einem prozentualen Gewichtsverhältnis von 10-20/40-45/40-45 im Terpolymer des Beschichtungsmaterials vorhanden sein.

Vorteilhaft kann das Terpolymer durch statistische Copolymerisation von Glykolid, ε-Caprolacton und Trimethylencarbonat hergestellt sein. Das Terpolymer kann ein mittleres Molekulargewicht im Bereich von mehr als 30000 Dalton aufweisen. Ferner kann das Terpolymer eine Glasübergangstemperatur im Bereich von -40 °C bis +20 °C aufweisen. Bevorzugt kann das Terpolymer eine Glasübergangstemperatur von -30 bis 0 °C aufweisen. Bedingt durch die amorphe Struktur und die niedrige Glasumwandlungstemperatur des Terpolymers ist die erfindungsgemässe Beschichtungszusammensetzung bei Raumtemperatur plastisch. Ein weiterer Vorteil eines Nahtmaterials, das erfindungsgemäss mit einem Terpolymer mit niedriger Glastemperatur beschichtet ist, ist dessen geringe Biegesteifigkeit und hohe Flexibilität.

Zum Vorteil der Eigenschaften des erfindungsgemässen Nahtmaterials kann das Beschichtungsmaterial, insbesondere das Polymer, eine inhärente Viskosität von 0,4 bis 3,0 dl/g, insbesondere von 0,7 bis 1,3 dl/g, gemessen in HFIP bei 25 θC und einer Konzentration von 0,5 Gew.-%, aufweisen.

Darüberhinaus kann das Beschichtungsmaterial mindestens einen mono- und/oder polyfunktionellen Alkohol enthalten. Insbesondere kann das Beschichtungsmaterial mono- und/oder polyfunktionellen Alkohol in einem Anteil von 0,02 bis 8 Gew.-% enthalten. Ferner kann das Beschichtungsmaterial mindestens eine mono- und/oder polyfunktionelle Carbonsäure, deren Anhydride und/oder Ester enthalten. Insbesondere kann das Beschichtungsmaterial mono- und/oder polyfunktionelle Carbonsäure und/oder deren Derivate in einem Anteil von 0,02 bis 8 Gew.-% enthalten. Bei der Herstellung des Terpolymers kann durch Zugabe von Molekulargewichtsreglern wie den vorstehend genannten Alkohol und/oder Carbonsäure sowie deren Derivaten das Molekulargewicht des Polymers verringert werden, um ausreichend plastisches Polymer, insbesondere ein wachsartiges Polymer zu erhalten. Durch geeignete Auswahl der Art und Menge des Molekulargewichtsreglers kann das Molekulargewicht in gewünschter Weise eingestellt werden.

Ferner kann Einmischen eines Weichmachers oder Plastifizierungsmittels in das Terpolymer gemäss der Erfindung die Plastizität noch weiter erhöhen. Bevorzugt sind dabei Weichmacher, die mit dem Terpolymer eine verträgliche Mischung bilden, ohne dass Phasenseparation eintritt. Als Beispiele für gemäss der Erfindung anwendbare Weichmacher sind Fette und Öle (z. B. Rizinusöl), deren Ester und Metallsalze, Glycerin, Diethylphthalate, Polyethylenglykol, Polypropylenglykol, Citrate und Phosphate zu nennen.

Die Beimischung eines Weichmachers, auch Blending genannt, kann zur noch heissen Polymerschmelze im direkten Anschluss an die Polymerisationsreaktion erfolgen oder in einem getrennten Verfahrensschritt. Dabei ist die thermische Stabilität des Weichmachers zu berücksichtigen. In einer bevorzugten Ausführungsform kann das Beschichtungsmittel mindestens ein Plastifizierungsmittel in einem Anteil von 1 bis 30 Gew.-% enthalten.

Erfindungsgemäss bevorzugt kann beim Nahtmaterial die Beschichtung aus einer Kombination des bioresorbierbaren Polymeren mit Fettsäuresalzen, insbesondere Calciumstearat und/oder Magnesiumstearat gebildet sein.

Das Beschichtungsmaterial kann einen sehr hohen Anteil am chirurgischen Nähfaden ausmachen. Im erfindungsgemässen Nahtmaterial kann das Beschichtungsmittel 0,2 bis 50 Gew.-% des Gesamtgewichts des beschichteten Nahtmaterials ausmachen.

Ein gemäss der Erfindung ausgebildetes Nahtmaterial zeichnet sich vorteilhaft durch einen guten Knotenhalt und gegenüber bekannten Nahtmaterialien verbessertem Knotenlaufverhalten aus.

Die vorliegende Erfindung betrifft ferner ein Beschichtungsmaterial für chirurgisches Nahtmaterial gebildet im wesentlichen aus einem bioresorbierbaren Polymer, insbesondere im wesentlichen gebildet aus dem Terpolymer wie es oben beschrieben ist. In einer bevorzugten Ausführungsform kann das Beschichtungsmaterial im wesentlichen aus einem wachsartigen bioresorbierbaren Polymer gebildet sein.

Mit Vorteil kann beim Beschichtungsmaterial das Terpolymer in fliessfähigem Zustand ohne Lösemittel, insbesondere geschmolzen, auf Nahtmaterial applizierbar sein.

In einer bevorzugten Ausführungsform kann das Beschichtungsmaterial aus einer Kombination des bioresorbierbaren Polymers mit Fettsäuresalzen, insbesondere Calciumstearat und/oder Magnesiumstearat gebildet sein.

Der Abbau der erfindungsgemässen Beschichtung für chirurgisches Nahtmaterial erfolgt im Körper eines Tieres oder eines Menschen durch Hydrolyse, an der Körper- und Gewebeflüssigkeiten beteiligt sind. Durch die Hydrolyse wird die Polymerkette in kleinere und leichter lösliche Fragmente gespalten. Die Bruchstücke werden gegebenenfalls unter Beteiligung von Makrophagen weiter abgebaut. Die Abbauprodukte werden durch das Stoffwechselsystem abtransportiert und wie andere Stoffwechselschlacken aus dem Organismus bzw. als Kohlendioxid und Wasser ausgeschieden. Für eine gute Verträglichkeit des beschichteten Nahtmaterials beim Patienten ist es wichtig, dass sich während des Abbauvorganges keine schädlichen Metaboliten bilden oder anreichern.

Polyglykolsäure zeichnet sich insbesondere dadurch aus, dass bei ihrer Zersetzung in vivo keine toxischen Zerfallsprodukte gebildet werden. Die erfindungsgemäss als Comonomere verwendeten Trimethylencarbonat und Caprolacton sind ebenfalls durch gute Verträglichkeit und Vermeidung toxischer Reaktionen gekennzeichnet. Das Degradationsverhalten des erfindungsgemässen Terpolymers kann durch Variation des Gesamtglykolidanteils im Polymer verändert werden, da Polyglykolsäure im Vergleich zu Polytrimethylencarbonat und Poly-ε-Caprolacton deutlich geringere Degradationszeiten aufweist.

Des weiteren betrifft die Erfindung ein Verfahren zur Herstellung eines Nahtmaterials für die Chirurgie bestehend aus einem oder mehreren Filamenten mit einer Beschichtung aus einem Terpolymer mit völlig amorpher Struktur wie es oben beschrieben ist, das dadurch gekennzeichnet ist, dass das Beschichten durch Aufbringen eines bioresorbierbaren Polymers vorgenommen wird, das im wesentlichen aus einem statistischen Terpolymer mit völlig amorpher Struktur gebildet ist.

Beim erfindungsgemässen Verfahren kann zum Beschichten des Nahtmaterials eine Lösung des Terpolymers aufgebracht werden, in der das Terpolymer gelöst in einem organischen Lösemittel ausgewählt aus der Gruppe der nicht toxischen organischen Lösemittel, der Ester, Ketone oder Mischungen davon verwendet wird. Als Beispiele für solche Lösemittel sind Ester wie Ethylacetat und andere Essigester, Ketone wie Aceton oder Lösemittelgemische zu nennen. Bevorzugt kann das Terpolymer für eine Lösung zum Beschichten in einer Konzentration von 0,1 bis 10 Gew.-%, insbesondere 0,5 bis 5 Gew.-% gelöst werden. Zur Anwendung vorbereitete Beschichtungslösungen zeichnen sich vorteilhaft durch Stabilität auch bei hohen Konzentrationen aus.

In einer besonderen Ausführungsform des Verfahrens kann das chirurgische Nahtmaterial zum Beschichten durch eine Lösung des Terpolymers hindurchgezogen werden. Mit Vorteil kann der Faden unter leichter Spannung durch das Beschichtungsbad geführt werden. Im allgemeinen kann der beschichtete Faden direkt nach Herausziehen aus der Lösung, ohne Zwischenbehandlung wie etwa Abstreifen, einem Trocknungsschritt zugeführt werden.

In einer anderen Ausführungsform des Verfahrens kann das Nahtmaterial zum Beschichten mit einer Lösung des Terpolymers besprüht werden.

In einer bevorzugten Ausführungsform kann zum Beschichten auf das Nahtmaterial eine Lösung des Terpolymers mittels eines Avivierstiftes aufbracht werden.

Ferner kann das Beschichten mittels einer oder mehrerer Auftragswalzen oder auf andere den Fachleuten bekannte Weise vorgenommen werden.

Besonders bevorzugt kann die Beschichtung bei einer Temperatur bis 40 °C, insbesondere bei Raumtemperatur durchgeführt werden. Mit Vorteil kann beim erfindungsgemässen Verfahren nach dem Auftragen der Beschichtung das Nahtmaterial mit einer Heizeinrichtung bei 80 bis 160 °C, insbesondere 130 °C getrocknet werden. Bevorzugt kann das Nahtmaterial berührungslos durch die Heizeinrichtung geführt werden. Das Nahtmaterial selbst wird dabei im allgemeinen nicht auf die Trocknungstemperatur erwärmt, um Schädigungen des Polymers zu vermeiden. Ebenfalls möglich ist ein Trocknen durch Anblasen mit erhitztem Gas, beispielsweise warmer Luft. Um Lösemittelreste zu entfernen, kann ein weiterer Trocknungsschritt bei vermindertem Druck, im Druckbereich weniger Millibar, vorgenommen werden.

Bei der Beschichtung mittels einer Lösung des Polymers wird das Lösemittel während des Trocknungsvorgangs aus der Beschichtung verdampft, so dass nach seiner Entfernung die Beschichtung im trockenen Zustand auf dem Nahtmaterial verbleibt. Auf diese Weise ergibt sich mit geringem technischen Aufwand, in kurzer Zeit und kostengünstig eine sehr gleichmässige Beschichtung.

In einer besonderen Ausführungsform des erfindungsgemässen Verfahrens kann die Beschichtung mit Terpolymer in fliessfähigem Zustand, insbesondere geschmolzen, vorgenommen werden.

In Weiterbildung kann sich das Verfahren gemäss der Erfindung dadurch auszeichnen, dass die Beschichtung mit einem bioresorbierbaren Polymer in Kombination mit Fettsäuresalzen, insbesondere Calciumstearat und/oder Magnesiumstearat vorgenommen wird.

Die vorliegende Erfindung betrifft ausserdem die Verwendung eines bioresorbierbaren Polymers, das im wesentlichen aus einem statistischen Terpolymer, wie es oben beschrieben ist, mit völlig amorpher Struktur gebildet ist, als Beschichtungsmittel auf Nahtmaterial für die Chirurgie.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

### Beispiel 1

250 g eines statistischen Terpolymers mit der Zusammensetzung Glycolid/Trimethylencarbonat/e-Caprolacton 10/45/45 Gew.-% werden in 9750 g Essigsäureethylester (Ethylacetat) unter Rühren gelöst. Die Lösung wird in einen Vorratsbehälter gegeben und durch einen auf 22 °C temperierten kleineren Behälter im Kreislauf gepumpt. Ein geflochtener chirurgischer Nähfaden der Stärke USP 2/0 aus Polyglykolsäure wird durch die Lösung im kleinen Behälter geleitet und mit dieser Lösung beschichtet. Unmittelbar danach wird der Faden durch einen Heizkanal geführt, der auf 140 °C temperiert ist, wobei das Lösemittel abdampft. Der verbleibende Rest an Lösemittel wird mit trockener Luft bei ca. 50 °C getrocknet. Auf dem glatten, homogen beschichteten Nahtmaterial verbleiben ca. 2 Gew.-% fester Bestandteile.

Der Nähfaden besitzt hervorragende Knotenlaufeigenschaften sowohl im trockenen als auch im feuchten Zustand. Ein beschichteter Nähfaden der Stärke USP 1 zeigt vergleichbare Eigenschaften.

### Beispiel 2

350 g eines statistischen Terpolymers mit der Zusammensetzung Glycolid/Trimethylencarbonat/e-Caprolacton 10/60/30 Gew.-% werden in 9650 g Essigsäureethylester (Ethylacetat) unter Rühren gelöst. Die Lösung wird in einen Vorratsbehälter gegeben und durch einen auf 22 °C temperierten kleineren Behälter im Kreislauf gepumpt. Ein geflochtener chirurgischer Nähfaden der Stärke USP 3/0 aus Polyglykolsäure wird durch die Lösung im kleinen Behälter geleitet und mit dieser Lösung beschichtet. Unmittelbar danach wird der Faden durch einen Heizkanal geführt, der auf 160 °C temperiert ist, wobei das Lösemittel abdampft. Der verbleibende Rest an Lösemittel wird mit trockener Luft bei ca. 50 °C getrocknet. Auf dem glatten, homogen beschichteten Faden verbleiben ca. 3 Gew.-% fester Bestandteile.

Das Nahtmaterial besitzt hervorragende Knotenlaufeigenschaften sowohl im trockenen als auch im feuchten Zustand. Ein beschichteter Nähfaden der Stärke USP 1 zeigt vergleichbare Eigenschaften.

### Beispiel 3

300 g eines statistischen Terpolymers mit der Zusammensetzung Glycolid/Trimethylencarbonat/e-Caprolacton 20/40/40 Gew.-% werden in 9700 g Aceton unter Rühren gelöst. Die Lösung wird in einen Vorratsbehälter gegeben und durch einen auf 25 °C temperierten kleineren Behälter im Kreislauf gepumpt. Ein geflochtener Faden der Stärke USP 2/0 aus Polyglykolsäure wird durch die Lösung im kleinen Behälter geleitet und mit dieser Lösung beschichtet. Unmittelbar danach wird der Faden durch einen Heizkanal geführt, der auf 160 °C temperiert ist, wobei das Lösemittel abdampft. Der verbleibende Rest an Lösemittel wird mit trockener Luft bei ca. 50 °C getrocknet. Auf dem glatten, homogen beschichteten Faden verbleiben ca. 3 Gew.-% fester Bestandteile.

Der Nähfaden besitzt hervorragende Knotenlaufeigenschaften sowohl im trockenen als auch im feuchten Zustand. Ein beschichteter Nähfaden der Stärke USP 1 zeigt vergleichbare Eigenschaften.

## Patentansprüche

1. Nahtmaterial für die Chirurgie bestehend aus einem oder mehreren Filamenten und ausgebildet mit einer Beschichtung, **dadurch gekennzeichnet, dass** die Beschichtung mindestens teilweise aus einem wachsartigen bioresorbierbaren Polymer besteht, das im wesentlichen aus einem statistischen Terpolymer mit völlig amorpher Struktur gebildet ist, das Terpolymer unter Verwendung von Glykolid, ε-Caprolacton und Trimethylencarbonat gebildet ist, insbesondere aus diesen besteht, und im Terpolymer Glykolid in einem Anteil von 5 bis 50 Gew.-%, ε-Caprolacton in einem Anteil von 5 bis 90 Gew.-% und Trimethylencarbonat in einem Anteil von 5 bis 90 Gew.-% vorhanden ist.

2. Nahtmaterial nach Anspruch 1, **dadurch gekennzeichnet, dass** im Terpolymer Trimethylencarbonat und ε-Caprolacton in einem Gewichtsverhältnis zwischen 95 : 5 und 5 : 95, insbesondere in einem Gewichtsverhältnis von 30 : 70 und 70 : 30, bevorzugt von 50 : 50 vorhanden sind.

3. Nahtmaterial nach einem der vorhergehenden Ansprüche, **dadurch gekennzeichnet, dass** das Terpolymer durch statistische Copolymerisation von Glykolid, ε-Caprolacton und Trimethylencarbonat hergestellt ist.

4. Nahtmaterial nach einem der vorhergehenden Ansprüche, **dadurch gekennzeichnet, dass** das Terpolymer ein mittleres Molekulargewicht im Bereich von mehr als 30000 Dalton aufweist.

5. Nahtmaterial nach einem der vorhergehenden Ansprüche, **dadurch gekennzeichnet, dass** das Terpolymer eine Glasübergangstemperatur im Bereich von -40 °C bis +20 °C aufweist.

6. Nahtmaterial nach einem der vorhergehenden Ansprüche, **dadurch gekennzeichnet, dass** das Beschichtungsmaterial, insbesondere das Polymer, eine inhärente Viskosität von 0,4 bis 3,0 dl/g, insbesondere von 0,7 bis 1,3 dl/g, gemessen in HFIP bei 25 °C und einer Konzentration von 0,5 Gew.-%, aufweist.

7. Nahtmaterial nach einem der vorhergehenden Ansprüche, **dadurch gekennzeichnet, dass** das Beschichtungsmaterial mindestens ein Plastifizierungsmittel in einem Anteil von 1 bis 30 Gew.-% enthält.

8. Nahtmaterial nach einem der vorhergehenden Ansprüche, **dadurch gekennzeichnet, dass** die Beschichtung aus einer Kombination des bioresorbierbaren Polymers mit Fettsäuresalzen, insbesondere Calciumstearat und/oder Magnesiumstearat gebildet ist.

9. Nahtmaterial nach einem der vorhergehenden Ansprüche, **dadurch gekennzeichnet, dass** die Beschichtung 0,2 bis 50 Gew.-% des Gesamtgewichts des beschichteten Nahtmaterials ausmacht.

10. Beschichtungsmaterial für chirurgisches Nahtmaterial für die Chirurgie gebildet im wesentlichen aus einem bioresorbierbaren Polymer, insbesondere im wesentlichen gebildet aus dem Terpolymer nach einem der vorhergehenden Ansprüche.

11. Beschichtungsmaterial nach Anspruch 10, **dadurch gekennzeichnet, dass** das Terpolymer in fliessfähigem Zustand ohne Lösemittel, insbesondere geschmolzen, auf Nahtmaterial applizierbar ist.

12. Beschichtungsmaterial nach Anspruch 10 oder 11, **dadurch gekennzeichnet, dass** es aus einer Kombination des bioresorbierbaren Polymers mit Fettsäuresalzen, insbesondere Calciumstearat und/oder Magnesiumstearat gebildet ist.

13. Verfahren zur Herstellung eines Nahtmaterials für die Chirurgie bestehend aus einem oder mehreren Filamenten mit einer Beschichtung aus einem statistischen Terpolymer nach einem der vorhergehenden Ansprüche mit völlig amorpher Struktur, **dadurch gekennzeichnet, dass** das Beschichten durch Aufbringen eines bioresorbierbaren Polymers vorgenommen wird, das im wesentlichen aus einem statistischen Terpolymer mit völlig amorpher Struktur gebildet ist.

14. Verfahren nach Anspruch 13, **dadurch gekennzeichnet, dass** zum Beschichten des Nahtmaterials eine Lösung des Terpolymers aufgebracht wird, in der das Terpolymer gelöst in einem organischen Lösemittel ausgewählt aus der Gruppe der nicht toxischen organischen Lösemittel, insbesondere der Ester, Ketone oder Mischungen davon verwendet wird.

15. Verfahren nach Anspruch 13 oder 14, **dadurch gekennzeichnet, dass** das Terpolymer für eine Lösung zum Beschichten in einer Konzentration von 0,1 bis 10 Gew.-%, insbesondere 0,5 bis 5 Gew.-% gelöst wird.

16. Verfahren nach einem der Ansprüche 13 bis 15, **dadurch gekennzeichnet, dass** das Nahtmaterial zum Beschichten durch eine Lösung des Terpolymers hindurchgezogen wird.

17. Verfahren nach einem der Ansprüche 13 bis 15, **dadurch gekennzeichnet, dass** das Nahtmaterial zum Beschichten mit einer Lösung des Terpolymers besprüht wird.

18. Verfahren nach einem der Ansprüche 13 bis 15, **dadurch gekennzeichnet, dass** zum Beschichten auf das Nahtmaterial eine Lösung des Terpolymers mittels eines Avivierstiftes aufbracht wird.

19. Verfahren nach einem der Ansprüche 13 bis 18, **dadurch gekennzeichnet, dass** die Beschichtung bei einer Temperatur bis 40 °C, insbesondere Raumtemperatur durchgeführt wird.

20. Verfahren nach einem der Ansprüche 13 bis 18, **dadurch gekennzeichnet, dass** nach dem Auftragen der Beschichtung das Nahtmaterial mit einer Heizeinrichtung bei 80 bis 160 °C getrocknet wird.

21. Verfahren nach Anspruch 13, **dadurch gekennzeichnet, dass** die Beschichtung mit Terpolymer in fliessfähigem Zustand, insbesondere geschmolzen, vorgenommen wird.

22. Verfahren nach einem der Ansprüche 13 bis 21, **dadurch gekennzeichnet, dass** die Beschichtung mit einem bioresorbierbaren Polymer in Kombination mit Fettsäuresalzen, insbesondere Calciumstearat und/oder Magnesiumstearat vorgenommen wird.

23. Verwendung eines bioresorbierbaren Polymers, das im wesentlichen aus einem statistischen Terpolymer nach einem der vorhergehenden Ansprüche mit völlig amorpher Struktur gebildet ist, als Beschichtungsmittel auf Nahtmaterial für die Chirurgie.

## Claims

1. Suture material for surgery comprising one or more filaments and formed with a coating, **characterized in that** the coating at least partly comprises a waxy bioabsorbable polymer, which is essentially formed from a random terpolymer with a completely amorphous structure, the terpolymer is formed using glycolide, ε-caprolactone and trimethylene carbonate, in particular comprises these, and the terpolymer contains glycolide in a proportion of 5 to 50 wt. %, ε-caprolactone in a proportion of 5 to 90 wt. % and trimethylene carbonate in a proportion of 5 to 90 wt. %.

2. Suture material according to Claim 1, **characterized in that** the terpolymer contains trimethylene carbonate and ε-caprolactone in a weight ratio between 95:5 and 5:95, in particular in a weight ratio of 30 : 70 and 70 : 30, preferably of 50 : 50.

3. Suture material according to any of the preceding claims, **characterized in that** the terpolymer is produced by random copolymerization of glycolide, ε-caprolactone and trimethylene carbonate.

4. Suture material according to any of the preceding claims, **characterized in that** the terpolymer has an average molecular weight in the range of more than 30,000 Dalton.

5. Suture material according to any of the preceding claims, **characterized in that** the terpolymer has a glass transition point in the range from -40 to +20°C.

6. Suture material according to any of the preceding claims, **characterized in that** the coating material, in particular the polymer, has an inherent viscosity of 0.4 to 3.0 dl/g, particularly 0.7 to 1.3 dl/g, measured in HFIP at 25°C and a concentration of 0.5 wt. %.

7. Suture material according to any of the preceding claims, **characterized in that** the coating material contains at least one plasticizer in a proportion of 1 to 30 wt. %

8. Suture material according to any of the preceding claims, **characterized in that** the coating is formed from a combination of the bioabsorbable polymer with fatty acid salts, in particular calcium stearate and/or magnesium stearate.

9. Suture material according to any of the preceding claims, **characterized in that** the coating represents 0.2 to 50 wt. % of the total weight of the coated suture material.

10. Coating material for surgical suture material for surgery essentially formed from a bioabsorbable polymer, in particular essentially formed from the terpolymer according to any of the preceding claims.

11. Coating material according to Claim 10, **characterized in that** the terpolymer can be applied in the fluid state without solvent, in particular in a melted state, to the suture material.

12. Coating material according to Claim 10 or 11, **characterized in that** it is formed from a combination of the bioabsorbable polymer with fatty acid salts, in particular calcium stearate and/or magnesium stearate.

13. Process for the production of a suture material for surgery comprising one or more filaments with a coating composed of a random terpolymer according to any of the preceding claims having a completely amorphous structure, **characterized in that** the coating takes place by the application of a bioabsorbable polymer, which is essentially formed from a random terpolymer with a completely amorphous structure.

14. Process according to Claim 13, **characterized in that** for coating the suture material a solution of the terpolymer is applied, in which the terpolymer is used dissolved in an organic solvent selected from the group of non-toxic organic solvents, in particular esters, ketones or mixtures thereof.

15. Process according to Claim 13 or 14, **characterized in that** the terpolymer for a coating solution is dissolved in a concentration of 0.1 to 10, particularly 0.5 to 5 wt. %.

16. Process according to any of Claims 13 to 15, **characterized in that** for coating purposes, the suture material is drawn through a solution of the terpolymer.

17. Process according to any of Claims 13 to 15, **characterized in that** for coating purposes the suture material is sprayed with a solution of the terpolymer.

18. Process according to any of Claims 13 to 15, **characterized in that** for coating purposes a solution of the terpolymer is applied to the suture material using a finishing stick.

19. Process according to any of Claims 13 to 18, **characterized in that** coating is performed at a temperature up to 40°C, particularly at room temperature.

20. Process according to any of Claims 13 to 18, **characterized in that** following the application of the coating, the suture material is dried with a heating device at 80 to 160°C.

21. Process according to Claim 13, **characterized in that** coating with the terpolymer takes place in the fluid state, particularly in the melted state.

22. Process according to any of Claims 13 to 21, **characterized in that** coating takes place with a bioabsorbable polymer combined with fatty acid salts, in particular calcium stearate and/or magnesium stearate.

23. Use of a bioabsorbable polymer which is essentially formed from a random terpolymer according to any of the preceding claims with a completely amorphous structure, as a coating agent on suture material for surgery.

## Revendications

1. Matériau de suture chirurgicale, constitué d'un ou de plus filaments et réalisé avec un revêtement, **caractérisé en ce que** le revêtement est constitué au moins en partie d'un polymère biorésorbable de type cire qui est formé essentiellement d'un terpolymère statistique à structure complètement amorphe, le terpolymère est transformé en recourant à du glycolide, de l'ε-caprolactone et du carbonate de triméthylène et étant en particulier constitué de ceux-ci, le glycolide étant présent dans le terpolymère à une teneur de 5 à 50 % en poids, l'ε-caprolactone à une teneur de 5 à 90 % en poids et le carbonate de triméthylène à une teneur de 5 à 90 % en poids.

2. Matériau de suture selon la revendication 1, **caractérisé en ce que** dans le terpolymère, le carbonate de triméthylène et l'ε-caprolactone sont présents dans un rapport pondéral compris entre 95 : 5 et 5 : 95, en particulier dans un rapport pondéral de 30 : 70 et 70 : 30 et de préférence dans un rapport pondéral de 50 : 50.

3. Matériau de suture selon l'une des revendications précédentes, **caractérisé en ce que** le terpolymère est préparé par copolymérisation statistique de glycolide, d'ε-caprolactone et de carbonate de triméthylène.

4. Matériau de suture selon l'une des revendications précédentes, **caractérisé en ce que** le terpolymère présente un poids moléculaire moyen de l'ordre de plus de 30 000 Dalton.

5. Matériau de suture selon l'une des revendications précédentes, **caractérisé en ce que** le terpolymère présente une température de transition vitreuse comprise dans la plage de -40°C à +20°C.

6. Matériau de suture selon l'une des revendications précédentes, **caractérisé en ce que** le matériau de revêtement et en particulier le polymère présente une viscosité intrinsèque de 0,4 à 3,0 dl/g et en particulier de 0,7 à 1,3 dl/g, mesurée dans le HFIP à 25°C et à une concentration de 0,5 % en poids.

7. Matériau de suture selon l'une des revendications précédentes, **caractérisé en ce que** le matériau de revêtement contient au moins un agent de plastification à une teneur de 1 à 30 % en poids.

8. Matériau de suture selon l'une des revendications précédentes, **caractérisé en ce que** le revêtement est formé d'une combinaison du polymère biorésorbable avec des sels d'acides gras, et en particulier le stéarate de calcium et/ou le stéarate de magnésium.

9. Matériau de suture selon l'une des revendications précédentes, **caractérisé en ce que** le revêtement représenté de 0,2 à 50 % en poids du poids total du matériau de suture revêtu.

10. Matériau de revêtement pour matériau de suture chirurgicale destiné à la chirurgie, constitué essentiellement d'un polymère biorésorbable et en particulier essentiellement constitué du terpolymère selon l'une des revendications précédentes.

11. Matériau de revêtement selon la revendication 10, **caractérisé en ce que** le terpolymère est appliqué sur le matériau de suture à l'état liquide sans solvant et en particulier à l'état fondu.

12. Matériau de revêtement selon les revendications 10 ou 11, **caractérisé en ce qu'**il est formé d'un combinaison du polymère biorésorbable et de sels d'acides gras et en particulier de stéarate de calcium et/ou de stéarate de magnésium.

13. Procédé de préparation d'un matériau de suture chirurgicale pour la chirurgie, constitué d'un ou de plusieurs filaments dotés d'un revêtement constitué d'un terpolymère statistique selon l'une des revendications précédentes et à structure complètement amorphe, **caractérisé en ce que** le revêtement est réalisé par application d'un polymère biorésorbable qui est formé essentiellement du terpolymère statistique à structure complètement amorphe.

14. Procédé selon la revendication 13, **caractérisé en ce que** pour revêtir le matériau de suture, on applique une solution du terpolymère dans laquelle le terpolymère est dissous dans un solvant organique sélectionné dans l'ensemble des solvants organiques non toxiques, et en particulier des esters, cétones ou mélanges de ceux-ci.

15. Procédé selon les revendications 13 ou 14, **caractérisé en ce que** le terpolymère est dissous à une concentration de 0,1 à 10 % en poids et en particulier de 0,5 % à 5 % en poids pour obtenir une solution de revêtement.

16. Procédé selon l'une des revendications 13 à 15, **caractérisé en ce que** le matériau de suture est passé dans une solution du terpolymère pour être revêtu.

17. Procédé selon l'une des revendications 13 à 15, **caractérisé en ce que** le matériau de suture est pulvérisé par une solution du terpolymère pour être revêtu.

18. Procédé selon l'une des revendications 13 à 15, **caractérisé en ce que**, pour revêtir le matériau de suture, on applique une solution du terpolymère au moyen d'une tige d'avivage.

19. Procédé selon l'une des revendications 13 à 18, **caractérisé en ce que** le revêtement est réalisé à une température de jusque 40°C et en particulier à la température ambiante.

20. Procédé selon l'une des revendications 13 à 18, **caractérisé en ce qu'**après l'application du revêtement, le matériau de suture est séché à l'aide d'un dispositif de chauffage réglé entre 80 et 160°C.

21. Procédé selon la revendication 13, **caractérisé en ce que** le revêtement est réalisé par le polymère à l'état liquide et en particulier à l'état fondu.

22. Procédé selon l'une des revendications 13 à 21, **caractérisé en ce que** le revêtement est réalisé avec un polymère biorésorbable combiné avec des sels d'acides gras et en particulier le stéarate de calcium et/ou le stéarate de magnésium.

23. Utilisation d'un polymère biorésorbable essentiellement constitué d'un terpolymère statistique selon l'un des revendications précédentes et à structure entièrement amorphe, comme agent de revêtement d'un matériau de suture chirurgicale.
